# EUROPEAN PATENT SPECIFICATION

(11) **EP 3 981 379 B1**
(45) Date of publication and mention of the grant of the patent: **31.12.2025**
(21) Application number: 21201252.0
(22) Date of filing: 06.10.2021
(51) Int. Cl.: A61K 8/06, A61K 8/45, A61K 8/86, A61K 8/9783, A61Q 19/08

(54) **A NANO-EMULSION COMPOSITION FOR SKIN CARE APPLICATION**
NANOEMULSIONSZUSAMMENSETZUNG ZUR HAUTPFLEGEANWENDUNG
COMPOSITION EN NANOÉMULSION POUR APPLICATION DE SOINS DE LA PEAU

(30) Priority: 07.10.2020 GB 202015902
(43) Date of publication of application: 13.04.2022
(73) Proprietor: Almora Botanica UK Ltd, London W1S 4LW (GB)
(72) Inventor: Kumar Rout, Deeleep, 560067 Bengaluru (IN); Kumar Patel, Neeraj, 560067 Bengaluru (IN); Ravi Varade, Shailesh, 440016 Nagpur (IN)
(74) Representative: Caldon, Giuliano

(56) References cited:
- WO-A1-2020/018512
- KR-B1- 102 034 142
- US-A1- 2019 314 255

## Description

### FIELD OF INVENTION

Embodiments of a present invention relates to emulsion composition, more particularly it relates to a nano-emulsion composition for skin care application.

### BACKGROUND

Emulsions are formulated to act as a moisture lock that seals in all the hydration and benefits of your essence and serums. The emulsions offer a lighter delivery method to moisturize skin and often are non-comedogenic. The emulsions also offer many of the same benefits of a cream, just in a lighter formula.

There are two basic types of emulsions: oil-in-water (O/W) and water-in-oil (W/O). In every emulsion there is a continuous phase that suspends the droplets of the other element which is called the dispersed phase.

Oil-in-water emulsions are conventionally defined as a thermodynamically unstable systems which include two immiscible liquids (generally water and oil), in which oil is distributed into the water. The oil-in-water Emulsions are used in moisturizing products and food products such as milk, vinaigrette and mayonnaise, o/w emulsions contain a low oil concentration. They are mixable with water, non-occlusive, non-greasy and will absorb water. The dispersion medium in these emulsions is water; o/w emulsifiers keep oil drops packed in water. For example, WO 2020/018512 A1 discloses stable nano-emulsions with an oil soluble phytoactive compound (cannabinoid oil), including the use of these in topical cosmetics.

However, emulsion have several disadvantages such as thermodynamically unstable, short shelf life, improper formulation of emulsions and improper selection of emulsifying agent. These parameters lead to phase inversion, creaming and cracking of the emulsion.

Hence, there is need for an emulsion composition with stable oil droplets over a wider temperature range.

### SUMMARY

In accordance with an embodiment of the invention, a nano-emulsion composition for skin care application according to the claims is provided. The nano-emulsion composition being oil-in-water composition. The nano-emulsion composition includes at least one oil present in a percentage of > 4 % by weight. The nano-emulsion composition includes at least one hydrophilic compound to create an aqueous phase. The at least one hydrophilic compound present in a percentage of 0.1 % to 15 % by weight. The nano-emulsion composition also includes at least one water soluble phytoactive compound present in a percentage of 0.5 % and 5 % by weight. The nano-emulsion composition includes at least one oil soluble phytoactive compound present in a percentage of 0.01 % and 5 % by weight. The nano-emulsion composition also includes a combination of polyglycerol fatty acid esters as emulsifiers. The combination of polyglycerol fatty acid esters comprise a first polyglycerol fatty acid ester and a second polyglycerol fatty acid ester in a ratio ranging from 70:30 to 40:60. The first polyglycerol fatty acid ester having number of carbon atoms and mean polymerisation degree represented by C₁₂G₁₀, and the second polyglycerol fatty acid ester having number of carbon atoms and mean polymerisation degree represented by C₁₈:₁G₁₀. The combination of polyglycerol fatty acid esters present in a percentage of 1 % and 10 % by weight. Further the nano-emulsion composition includes at least one preservative present in a percentage of 0.4 % to 4 % by weight. The nano-emulsion composition being a low viscous fluidic vehicle to be used as Serum, Micellar Water, Facial Cleanser etc. The nano-emulsion composition being capable of retaining high storage stability over a wider temperature range.

In accordance with another embodiment of the invention, a skin care composition comprising the nano-emulsion composition is provided.

In accordance with yet another embodiment of the invention, a cosmetic comprising the nano-emulsion composition is provided.

To further clarify the advantages and features of the present disclosure, a more particular description of the disclosure will follow by reference to specific embodiments thereof.

### DETAILED DESCRIPTION

For the purpose of promoting an understanding of the principles of the disclosure, reference will now be made to particular embodiments and specific language will be used to describe them. It will nevertheless be understood that no limitation of the scope of the disclosure is thereby intended. Such alterations and further modifications in the illustrated biosynthesis of magnesium oxide nanostructures, and such further applications of the principles of the disclosure as would normally occur to those skilled in the art are to be construed as being within the scope of the present disclosure.

The terms "comprises", "comprising", or any other variations thereof, are intended to cover a non-exclusive inclusion, such that a process or method that comprises a list of steps does not include only those steps but may include other steps not expressly listed or inherent to such a process or method. Similarly, one or more components, compounds, and ingredients preceded by "comprises... a" does not, without more constraints, preclude the existence of other components or compounds or ingredients or additional components. Appearances of the phrase "in an embodiment", "in another embodiment" and similar language throughout this specification may, but not necessarily do, all refer to the same embodiment.

Unless otherwise defined, all technical and scientific terms used herein have the same meaning as commonly understood by those skilled in the art to which this disclosure belongs. The system, methods, and examples provided herein are only illustrative and not intended to be limiting.

In the following specification and the claims, reference will be made to a number of terms, which shall be defined to have the following meanings. The singular forms "a", "an", and "the" include plural references unless the context clearly dictates otherwise.

Embodiments of the present invention relates to a nano-emulsion composition for skin care application.

As used herein the term 'nano-emulsion composition' relates to a colloidal system consisting of mainly oil, surfactant and water with high kinetic stability, low viscosity and is optically transparent.

The nano-emulsion composition mainly comprises oil-in-water emulsion composition for skin care application. The nano-emulsion composition being oil in water emulsion composition with a transparency >75 % for visible light. An oil droplets present in the nano-emulsion composition are of nano sized with many phytoactives solubilised in them for skin care benefits. The oil droplets are mono-dispersed in the nano-emulsion composition, thereby causing a good active transport into targeted site such as epidermis-dermis junction in the skin. The invention provides a stable, homogeneous, aqueous fluid product incorporating water-insoluble phytoactives into the nano-emulsion composition. The invention also provides an emollient which exhibits silky feel to the skin by virtue of proprietary oil incorporation into the nano-emulsion composition. The nano-emulsion composition being a low viscous fluidic vehicle to be used as Serum, Micellar Water, Facial Cleanser etc. The nano-emulsion composition being capable of retaining high storage stability over a wider temperature range.

In an embodiment, the nano-emulsion composition includes at least one oil. The at least one oil is present in a percentage of > 4 % by weight, based on total weight of the nano-emulsion composition. When the at least one oil present in this range, provides emollience during application of the skin care product using the nano-emulsion composition. The at least one oil also contributes to solubilising the phytoactive in the nano-emulsion composition.

The at least one oil for use in the invention is a kind of oil agent usually used for skin product preparations such as cosmetics. In an embodiment, the such at least one oil include hydrocarbons, fatty esters, fatty carbonates and triglycerides. The at least one oil being used possess wide physicochemical characteristics.

In another embodiment, the at least one oil is selected from a group consisting C15-19 Alkane, Undecane and Tridecane, Capric Caprylic Triglyceride, Coco caprylate or Coco caprate, Dicaprylyl carbonate, Cetyl Ricinoleate, and Isoamyl Cocoate. Any one of the aforementioned oils or combinations thereof may be used in the nano-emulsion composition for skin care application. The detail of the said group is provided in Table 1.

**Table 1**

| **INCI Name** | **Trade Name** | **Supplier** | **Origin** |
|---|---|---|---|
| C15-19 Alkane | Emogreen | SEPPIC | France |
| Undecane and Tridecane | Cetiol Ultimate | BASF | Germany |
| Capric Caprylic Triglyceride | Radia7104 | Evonik | UK |
| Cococaprylate | Cetiol C5 | BASF | Germany |
| Dicaprylyl Carbonate | Cetiol CC | BASF | Germany |
| Cetyl Ricinoleate | Tegosoft CR | Evonik | UK |
| Isoamyl Cocoate | Tegosoft AC | Evonik | UK |

The at least one oil being configured to function as a dispersed phase in the oil-in-water nano-emulsion composition. The at least one oil comprises the oil droplets of size 100 -300 nm. The oil droplets present in the nano-emulsion composition include many phytoactives solubilised in them for skin care benefits. The oil droplets are mono-dispersed in the nano-emulsion composition, thereby causing a good active transport into targeted site such as epidermis-dermis junction in the skin.

In an embodiment, the nano-emulsion composition includes at least one hydrophilic compound to create an aqueous phase. The at least one hydrophilic compound is present in a percentage of between 0.1 % and 15 %, preferably 10% by weight, based on total weight of the nano-emulsion composition. The at least one hydrophilic compound configured to function as a continuous phase in the oil-in-water nano-emulsion composition.

The at least one hydrophilic compound is selected from a group comprising of glycerol, levulinic acid, sorbic acid and their potassium or sodium salts, phytols, polyphenols, plant extracts, biopolysaccharides, sugars and emulsifiers.

In an embodiment, the nano-emulsion composition includes at least one water soluble phytoactive compound. The at least one water soluble phytoactive compound is present in a percentage of between 0.5 % and 5 % by weight, preferably 1.5 % by weight, based on total weight of the nano-emulsion composition.

The at least one water soluble phytoactive compound is selected from a group comprising of *Glycyrrhiza Glabra* extract, *Mango* fruit extract, *Moris Alba* root extract, poppy seed extract, *Centella Asiatica* leaf extract and forsythoside B from plant extract.

The at least one water soluble phytoactive compound exhibit good skin care benefits. As according to the National Cancer Institute *Glycyrrhiza Glabra* extract has been used as an anti-inflammatory and antioxidant, primarily because of licochalcone, a molecule contained in liquorice root extract which helps control oil production and helps calm and soothe acne-prone skin.

The *Mango* fruit extract exhibit hydrating properties, as mangoes are full of antioxidants which is an amazing property in keeping the skin nourished, improving skin tone, providing healthy skin, preventing acne breakouts, soothing skin, promoting collagen production and brightening skin.

The *Moris Alba* root extract's main benefit for skin being calming signs of irritation along with its potential to fade the look of discolorations and uneven skin tone.

The poppy seed extract helps to restore the natural barrier and reduces trans epidermal water loss. The poppy seed extract is a natural emollient. The poppy seed extract also helps to soften and conditioning the hair whilst locking in moisture, hydrating and improving hair health and providing a non-greasy shine.

The *Centella Asiatica* leaf extract boosts antioxidant activity at the site of wounds, strengthening the skin and increasing blood circulation. The *Centella Asiatica* leaf extract is beneficial in the management of eczema, psoriasis, veins, and stretch marks. For acne sufferers, the *Centella Asiatica* leaf extract speeds healing, helping to prevent scarring and prevent future blemishes.

The forsythoside B from plant extract protects against the entry of particulate matter into the skin, removes and neutralizes damaging free radicals, strengthens the skin's barrier function by 18.6% versus control after exposure to particulate matter, repairs cell metabolism by boosting adenosine triphosphate (ATP) production.

In an embodiment, the nano-emulsion composition includes at least one oil soluble phytoactive compound. The at least one oil soluble phytoactive compound is present in a percentage of between 0.01 % and 5 % by weight, preferably 0.2 % by weight, based on total weight of the nano-emulsion composition.

The at least one oil soluble phytoactive compound is selected from a group comprising of phytol extracted from plant, boswellin and NG shea unsaponifiable.

The phytol extracted from plant reduces signs of skin aging of the face and body by stimulating adipogenesis and lipogenesis, and further stimulating collagen, elastin and extracellular matrix production. The boswellin has a skin-soothing effect.

The NG shea unsaponifiable soothes inflamed lips and rebuilds the lip integrity by strengthening the epidermis, restoring the barrier function and improving the moisture level. The NG shea unsaponifiable repairs damaged lips, making lips comfortable, soft and evenly pink.

In an embodiment, the nano-emulsion composition includes a combination of polyglycerol fatty acid esters as emulsifiers. The combination of polyglycerol fatty acid esters comprise a first polyglycerol fatty acid ester and a second polyglycerol fatty acid ester in a ratio ranging from 70:30 to 40:60. The first polyglycerol fatty acid ester having number of carbon atoms and mean polymerisation degree represented by C₁₂G₁₀ , and the second polyglycerol fatty acid ester having number of carbon atoms and mean polymerisation degree represented by C₁₈:₁G₁₀.

In an exemplary embodiment, the combination of polyglycerol fatty acid esters comprises the first polyglycerol fatty acid ester and the second polyglycerol fatty acid ester preferably in a ratio of 50:50.

In another exemplary embodiment, the combination of polyglycerol fatty acid esters comprises the first polyglycerol fatty acid ester and the second polyglycerol fatty acid ester preferably in a ratio of 60:40.

In yet another exemplary embodiment, polyglycerol-10 laurate (C₁₂G₁₀) and polyglycerol-10 oleate or polyglyceryl-10 oleate (C₁₈:₁G₁₀) being used as the first polyglycerol fatty acid ester and the second polyglycerol fatty acid ester, respectively. The polyglyceryl-10 laurate functions as a skin-softening emulsifier and cleansing agent. The polyglyceryl-10 oleate is an ester of decaglycerol and oleic acid and functions as a hydrating ingredient and emulsifier.

The combination of polyglycerol fatty acid esters is present in a percentage of between 1 % and 10 % by weight, preferably 4 % by weight, based on total weight of the nano-emulsion composition.

As used herein the term 'emulsifier' refers to a substance that stabilizes an emulsion, in particular an additive used to stabilize the creams, skin care products and processed food.

Further in an embodiment, the nano-emulsion composition includes at least one preservative. The at least one preservative is present in a percentage of between 0.5 % and 4 % by weight, preferably 1.5 % by weight, based on total weight of the nano-emulsion composition. The at least one preservative is selected from a group comprising of sodium levulinate, potassium sorbate and anisic acid.

In another embodiment of the present invention, a skin care composition comprising the nano-emulsion composition is provided. The skin care composition can be prepared by blending the above components and then dissolving, mixing or dispersing them by a conventional method.

In yet another embodiment of the present invention, a cosmetic comprising the nano-emulsion composition is provided. The cosmetic can be prepared by blending the above components and then dissolving, mixing or dispersing them by a conventional method according to the embodiment of the cosmetic.

In the present invention different ratios of the first polyglycerol fatty acid ester (C₁₂G₁₀) and the second polyglycerol fatty acid ester (C₁₈:₁G₁₀) were analysed to study optimum characteristics of the emulsion composition.
Table 2 enlists the different ratios of the C₁₂G₁₀ and the C₁₈:₁G₁₀ and characteristics studied.

**Table 2**

| **Ratio of C₁₂G₁₀ and C₁₈:₁G₁₀ in emulsion Composition** | **Emulsion Characteristics** | **% T** |
|---|---|---|
| 70:30 | Cloudy | <40 % |
| 60:40 | Clear | > 60 % but < 75 % |
| 50:50 | Clear | > 85 % |
| 40:60 | Cloudy | <40 % |
| 30:70 | Cloudy | <20 % |

According to analysis the emulsion composition comprising the C₁₂G₁₀ and the C₁₈:₁G₁₀ in the ratio of 50:50 by weight (1:1) exhibits optimum characteristics i.e. clear emulsion composition with > 85 % transparency (T). The emulsion composition comprising the C₁₂G₁₀ and the C₁₈:₁G₁₀ in the ratio of 60:40 by weight (3:2) also exhibits clear emulsion composition with > 60% but < 75 % transparency.

The present invention provides the nano-emulsion composition for skin care application and cosmetics. The nano-emulsion composition being oil in water emulsion composition with a transparency >75 % for visible light. The invention provides a stable, homogeneous, aqueous fluid product incorporating water-insoluble phytoactives into the nano-emulsion composition. The invention also provides an emollient which exhibits silky feel to the skin by virtue of proprietary oil incorporation into the nano-emulsion composition. The nano-emulsion composition being a low viscous fluidic vehicle to be used as Serum, Micellar Water, Facial Cleanser etc. The COSMetic Organic and Natural Standard (COSMOS) has certified the nano-emulsion composition being natural. The nano-emulsion composition being capable of retaining high storage stability over a wider temperature range.

## Claims

1. An oil-in-water nano-emulsion composition for skin care application, comprising:
at least one oil;
at least one hydrophilic compound;
at least one water soluble phytoactive compound;
at least one oil soluble phytoactive compound; and
a combination of polyglycerol fatty acid esters as emulsifiers,
wherein the combination of polyglycerol fatty acid esters comprises a first polyglycerol fatty acid ester and a second polyglycerol fatty acid ester in a ratio ranging from 70:30 to 40:60, wherein the first polyglycerol fatty acid ester having number of carbon atoms and mean polymerisation degree represented by C₁₂G₁₀, and the second polyglycerol fatty acid ester having number of carbon atoms and mean polymerisation degree represented by C₁₈:₁G₁₀;
wherein the at least one oil is present in an amount of > 4 % by weight, based on total weight of the nano-emulsion composition;
wherein the at least one hydrophilic compound is present in an amount between 0.1 % and 15 % by weight, based on total weight of the nano-emulsion composition;
wherein the at least one water soluble phytoactive compound is present in an amount between 0.5 % and 5 % by weight, based on total weight of the nano-emulsion composition;
wherein the at least one oil soluble phytoactive compound is present in an amount between 0.01 % and 5 % by weight, based on total weight of the nano-emulsion composition;
wherein the combination of polyglycerol fatty acid esters is present in an amount between 1 % and 10 % by weight, based on total weight of the nano-emulsion composition;
wherein the nano-emulsion composition further includes at least one preservative, which is present in a percentage of between 0.5 % and 4 % by weight, based on total weight of the nano-emulsion composition;
wherein the at least one oil is configured as a dispersed phase in the oil-in-water nano-emulsion composition, and comprises oil droplets of size 100-300 nm; wherein the oil droplets include phytoactives solubilised in them and are mono-dispersed in the oil-in-water nano-emulsion composition.

2. The nano-emulsion composition as claimed in claim 1, wherein combination of polyglycerol fatty acid esters comprises the first polyglycerol fatty acid ester and the second polyglycerol fatty acid ester preferably in a ratio of 50:50.

3. The nano-emulsion composition as claimed in claim 1, wherein combination of polyglycerol fatty acid esters comprises the first polyglycerol fatty acid ester and the second polyglycerol fatty acid ester preferably in a ratio of 60:40.

4. The composition as claimed in claim 1, wherein the nano-emulsion composition being oil in water emulsion composition with a transparency >75 % for visible light.

5. The composition as claimed in claim 1, wherein the at least one oil is selected from a group consisting hydrocarbons, fatty esters, fatty carbonates and triglycerides.

6. The composition as claimed in claim 1, wherein the at least one oil is selected from a group consisting C15-19 Alkane, Undecane and Tridecane, Capric Caprylic Triglyceride, Coco caprylate or Coco caprate, Dicaprylyl carbonate, Cetyl Ricinoleate, and Isoamyl Cocoate.

7. The composition as claimed in claim 1, wherein the at least one hydrophilic compound is selected from a group comprising of glycerol, levulinic acid, sorbic acid and their potassium or sodium salts, phytols, polyphenols, plant extracts, biopolysaccharides, sugars and emulsifiers.

8. The composition as claimed in claim 1, wherein the at least one water soluble phytoactive compound is selected from a group comprising of *Glycyrrhiza Glabra* extract, Mango fruit extract, *Moris Alba* root extract, poppy seed extract, *Centella Asiatica* leaf extract and forsythoside B from plant extract.

9. The composition as claimed in claim 1, wherein the at least one oil soluble phytoactive compound is selected from a group comprising phytol derived from plant extract, boswellin and NG shea unsaponifiable.

10. A skin care composition, comprising the nano-emulsion composition of any one of claims 1 to 9.

## Patentansprüche

1. Öl-in-Wasser-Nanoemulsionszusammensetzung zur Hautpflegeanwendung, Zusammensetzung:
mindestens ein Öl;
mindestens eine hydrophile Verbindung;
mindestens eine wasserlösliche phytoaktive Verbindung;
mindestens eine öllösliche phytoaktive Verbindung; und
eine Kombination von Polyglycerin-Fettsäureestern als Emulgatoren,
wobei die Kombination von Polyglycerin-Fettsäureestern einen ersten Polyglycerin-Fettsäureester und einen zweiten Polyglycerin-Fettsäureester in einem Verhältnis im Bereich von 70:30 bis 40:60 umfasst, wobei der erste Polyglycerin-Fettsäureester eine Anzahl von Kohlenstoffatomen und einen mittleren Polymerisationsgrad aufweist, der von C₁₂G₁₀ repräsentiert wird, und der zweite Polyglycerin-Fettsäureester eine Anzahl von Kohlenstoffatomen und einen mittleren Polymerisationsgrad aufweist, der von C₁₈:₁G₁₀ repräsentiert wird;
wobei das mindestens eine Öl in einer Menge von > 4 Gew.-%, bezogen auf das Gesamtgewicht der Nanoemulsionszusammensetzung, vorhanden ist;
wobei die mindestens eine hydrophile Verbindung in einer Menge zwischen 0,1 Gew.-% und 15 Gew.-%, bezogen auf das Gesamtgewicht der Nanoemulsionszusammensetzung, vorhanden ist;
wobei die mindestens eine wasserlösliche phytoaktive Verbindung in einer Menge zwischen 0,5 Gew.-% und 5 Gew.-%, bezogen auf das Gesamtgewicht der Nanoemulsionszusammensetzung, vorhanden ist;
wobei die mindestens eine öllösliche phytoaktive Verbindung in einer Menge zwischen 0,01 Gew.-% und 5 Gew.-%, bezogen auf das Gesamtgewicht der Nanoemulsionszusammensetzung, vorhanden ist;
wobei die Kombination von Polyglycerin-Fettsäureestern in einer Menge zwischen 1 Gew.-% und 10 Gew.-%, bezogen auf das Gesamtgewicht der Nanoemulsionszusammensetzung, vorhanden ist;
wobei die Nanoemulsionszusammensetzung außerdem mindestens ein Konservierungsmittel enthält, das in einem Prozentsatz zwischen 0,5 Gew.-% und 4 Gew.-%, bezogen auf das Gesamtgewicht der Nanoemulsionszusammensetzung, vorhanden ist;
wobei das mindestens eine Öl als dispergierte Phase in der Öl-in-Wasser-Nanoemulsionszusammensetzung vorliegt und Öltröpfchen mit einer Größe von 100 nm bis 300 nm umfasst; wobei die Öltröpfchen phytoaktive Stoffe enthalten, die in ihnen gelöst sind und in der Öl-in-Wasser-Nanoemulsionszusammensetzung monodispers vorliegen.

2. Nanoemulsionszusammensetzung nach Anspruch 1, wobei die Kombination von Polyglycerin-Fettsäureestern den ersten Polyglycerin-Fettsäureester und den zweiten Polyglycerin-Fettsäureester vorzugsweise in einem Verhältnis von 50:50 umfasst.

3. Nanoemulsionszusammensetzung nach Anspruch 1, wobei die Kombination von Polyglycerin-Fettsäureestern den ersten Polyglycerin-Fettsäureester und den zweiten Polyglycerin-Fettsäureester vorzugsweise in einem Verhältnis von 60:40 umfasst.

4. Zusammensetzung nach Anspruch 1, wobei die Nanoemulsionszusammensetzung eine Öl-in-Wasser-Emulsionszusammensetzung mit einer Transparenz von >75 % für sichtbares Licht ist.

5. Zusammensetzung nach Anspruch 1, wobei das mindestens eine Öl aus einer Gruppe ausgewählt wird, die aus Kohlenwasserstoffen, Fettsäureestern, Fettsäurecarbonaten und Triglyceriden besteht.

6. Zusammensetzung nach Anspruch 1, wobei das mindestens eine Öl aus einer Gruppe ausgewählt wird, die aus C15-19-Alkanen, Undecan und Tridecan, Caprylic/Capric Triglyceride, Coco-Caprylate/Caprate, Dicaprylylcarbonat, Cetyl-Ricinoleat und Isoamyl-Cocoate besteht.

7. Zusammensetzung nach Anspruch 1, wobei die mindestens eine hydrophile Verbindung aus einer Gruppe ausgewählt wird, die aus Glycerin, Lävulinsäure, Sorbinsäure und ihren Kalium- oder Natriumsalzen, Phytolen, Polyphenolen, Pflanzenextrakten, Biopolysacchariden, Zuckern und Emulgatoren besteht.

8. Zusammensetzung nach Anspruch 1, wobei die mindestens eine wasserlösliche phytoaktive Verbindung aus einer Gruppe ausgewählt wird, die Extrakt von *Glycyrrhiza Glabra,* Mangofruchtextrakt, *Moris-Alba*-Wurzelextrakt, Mohnsamenextrakt, *Centella-Asiatica*-Blattextrakt und Forsythosid B aus Pflanzenextrakt umfasst.

9. Zusammensetzung nach Anspruch 1, wobei die mindestens eine öllösliche phytoaktive Verbindung aus einer Gruppe ausgewählt wird, die aus Pflanzenextrakt gewonnenes Phytol, Boswellin und unverseifbares NG-Shea umfasst.

10. Hautpflegezusammensetzung, umfassend die Nanoemulsionszusammensetzung nach einem beliebigen der Ansprüche 1 bis 9.

## Revendications

1. Composition en nano-émulsion huile-dans-l'eau pour l'application de soins de la peau, composition:
au moins une huile ;
au moins un composé hydrophile ;
au moins un composé phytoactif soluble dans l'eau ;
au moins un composé phytoactif soluble dans l'huile ; et
une combinaison d'esters d'acides gras de polyglycérol comme émulsifiants,
où la combinaison d'esters d'acide gras de polyglycérol comprend un premier ester d'acide gras de polyglycérol et un deuxième ester d'acide gras de polyglycérol dans un rapport allant de 70:30 à 40:60, le premier ester d'acide gras de polyglycérol ayant un nombre d'atomes de carbone et un degré de polymérisation moyen représentés par C₁₂G₁₀, et le deuxième ester d'acide gras de polyglycérol ayant un nombre d'atomes de carbone et un degré de polymérisation moyen représentés par C₁₈:₁G₁₀;
où l'au moins une huile est présente dans une quantité de > 4 % en poids, sur la base du poids total de la composition en nano-émulsion ;
où l'au moins un composé hydrophile est présent dans une quantité comprise entre 0,1 % et 15 % en poids, sur la base du poids total de la composition en nano-émulsion ;
où l'au moins un composé phytoactif soluble dans l'eau est présent dans une quantité comprise entre 0,5 % et 5 % en poids, sur la base du poids total de la composition en nano-émulsion ;
où l'au moins un composé phytoactif soluble dans l'huile est présent dans une quantité comprise entre 0,01 % et 5 % en poids, sur la base du poids total de la composition en nano-émulsion ;
où la combinaison d'esters d'acide gras de polyglycérol est présente dans une quantité comprise entre 1 % et 10 % en poids, sur la base du poids total de la composition en nano-émulsion ;
où la composition en nano-émulsion comprend en outre au moins un conservateur, qui est présent dans un pourcentage compris entre 0,5 % et 4 % en poids, sur la base du poids total de la composition en nano-émulsion ;
où l'au moins une huile est configurée comme une phase dispersée dans la composition en nano-émulsion huile-dans-l'eau, et comprend des gouttelettes d'huile d'une taille de 100 à 300 nm ; où les gouttelettes d'huile comprennent des phytoactifs solubilisés dans celles-ci et sont mono-dispersées dans la composition en nano-émulsion huile-dans-l'eau.

2. Composition en nano-émulsion selon la revendication 1, où la combinaison d'esters d'acide gras de polyglycérol comprend le premier ester d'acide gras de polyglycérol et le deuxième ester d'acide gras de polyglycérol, de préférence dans un rapport de 50:50.

3. Composition en nano-émulsion selon la revendication 1, où la combinaison d'esters d'acide gras de polyglycérol comprend le premier ester d'acide gras de polyglycérol et le deuxième ester d'acide gras de polyglycérol, de préférence dans un rapport de 60:40.

4. Composition selon la revendication 1, où la composition en nano-émulsion est une composition d'émulsion huile-dans-l'eau avec une transparence >75 % pour la lumière visible.

5. Composition selon la revendication 1, où l'au moins une huile est choisie dans un groupe constitué d'hydrocarbures, d'esters gras, de carbonates gras et de triglycérides.

6. Composition selon la revendication 1, où l'au moins une huile est choisie dans un groupe constitué d'alcanes C15-19, d'undécane et de tridécane, de triglycérides capryliques-capriques, de caprylate de coco ou de caprate de coco, de carbonate de dicaprylyle, de ricinoléate de cétyle et de cocoate d'isoamyle.

7. Composition selon la revendication 1, où l'au moins un composé hydrophile est choisi dans un groupe comprenant le glycérol, l'acide lévulinique, l'acide sorbique et leurs sels de potassium ou de sodium, les phytols, les polyphénols, les extraits de plantes, les biopolysaccharides, les sucres et les émulsifiants.

8. Composition selon la revendication 1, où l'au moins un composé phytoactif soluble dans l'eau est choisi dans un groupe comprenant un extrait de *Glycyrrhiza Glabra,* un extrait de fruit de mangue, un extrait de racine *Moris Alba,* un extrait de graine de pavot, un extrait de feuille *Centella Asiatica* et du forsythoside B d'un extrait végétal.

9. Composition selon la revendication 1, où l'au moins un composé phytoactif soluble dans l'huile est choisi dans un groupe comprenant du phytol dérivé d'un extrait végétal, de la boswelline et de l'insaponifiable de karité NG.

10. Composition de soins de la peau, comprenant la composition en nano-émulsion selon l'une quelconque des revendications 1 à 9.
